(19) Europäisches Patentamt
European Patent Office
Office européen des brevets

(11) Publication number: 0 262 305 B1

(12) # EUROPEAN PATENT SPECIFICATION

(45) Date of publication of the patent specification: 31.10.90

(51) Int. Cl.[5]: C07C 229/44, C07D 215/54, C07C 69/716

(21) Application number: 87108676.5

(22) Date of filing: 16.06.87

(54) **Method for the preparation of anilinofumarate and quinoline-2,3-dicarboxylic acid.**

(30) Priority: 29.08.86 US 902274
29.08.86 US 902275

(43) Date of publication of application: 06.04.88 Bulletin 88/14

(45) Publication of the grant of the patent: 31.10.90 Bulletin 90/44

(84) Designated Contracting States: AT BE CH DE ES FR GB GR IT LI LU NL SE

(56) References cited:
DE-A- 2 942 105
GB-A- 1 270 413
US-A- 4 459 409

The file contains technical information submitted after the application was filed and not included in this specification

(73) Proprietor: AMERICAN CYANAMID COMPANY, 1937 West Main Street P.O. Box 60, Stamford Connecticut 06904-0060(US)

(72) Inventor: Maulding, Donald Roy, 57 Katydid Drive, Somerville New Jersey 08876(US)

(74) Representative: Wächtershäuser, Günter, Dr., Tal 29, D-8000 München 2(DE)

EP 0 262 305 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

The present invention relates to novel methods for preparing quinoline-2,3-dicarboxylic acids. These acids are useful intermediates in the preparation of herbicidal pyridine and quinoline imidazolinone herbicidal compounds.

The herbicidal pyridine and quinoline imidazolinone compounds prepared from the present compounds include 2-(4-isopropyl-4-methyl-5-oxo-2-imidazolin-2-yl)quinoline-3-carboxylic acid, esters and salts thereof and are disclosed in European Patent Application 0 041 623, (incorporated herein by reference). These herbicidal imidazolinyl quinoline-carboxylic acids may be prepared by the procedure described in United States Patent 4,518,780 (incorporated herein by reference) by cyclization, under basic conditions, an appropriately substituted 2-carbamoyl quinoline-3-carboxylic acid that, in turn, is prepared by the reaction of a substituted quinoline-2,3-dicarboxylic acid anhydride and appropriately substituted aminocarboxamide or aminothiocarboxamide. Quinoline-2,3-dicarboxylic acid anhydrides are prepared from the diacids by procedures well known in the art. However, the diacids themselves are not readily available.

A method useful for the preparation of quinoline-2,3-dicarboxylic acid and esters thereof by reacting a beta-anilino-alpha,beta-unsaturated ester with an immonium salt (commonly called a Vilsmeier reagent) is available in a co-pending application. The beta-anilino-alpha,beta-unsaturated esters are obtained by the reaction of appropriately substituted anilines with keto-esters or dialkyl acetylene dicarboxylates. This overall reaction for the preparation of quinoline-2,3-dicarboxylates is illustrated in Flow Diagram I.

FLOW DIAGRAM I

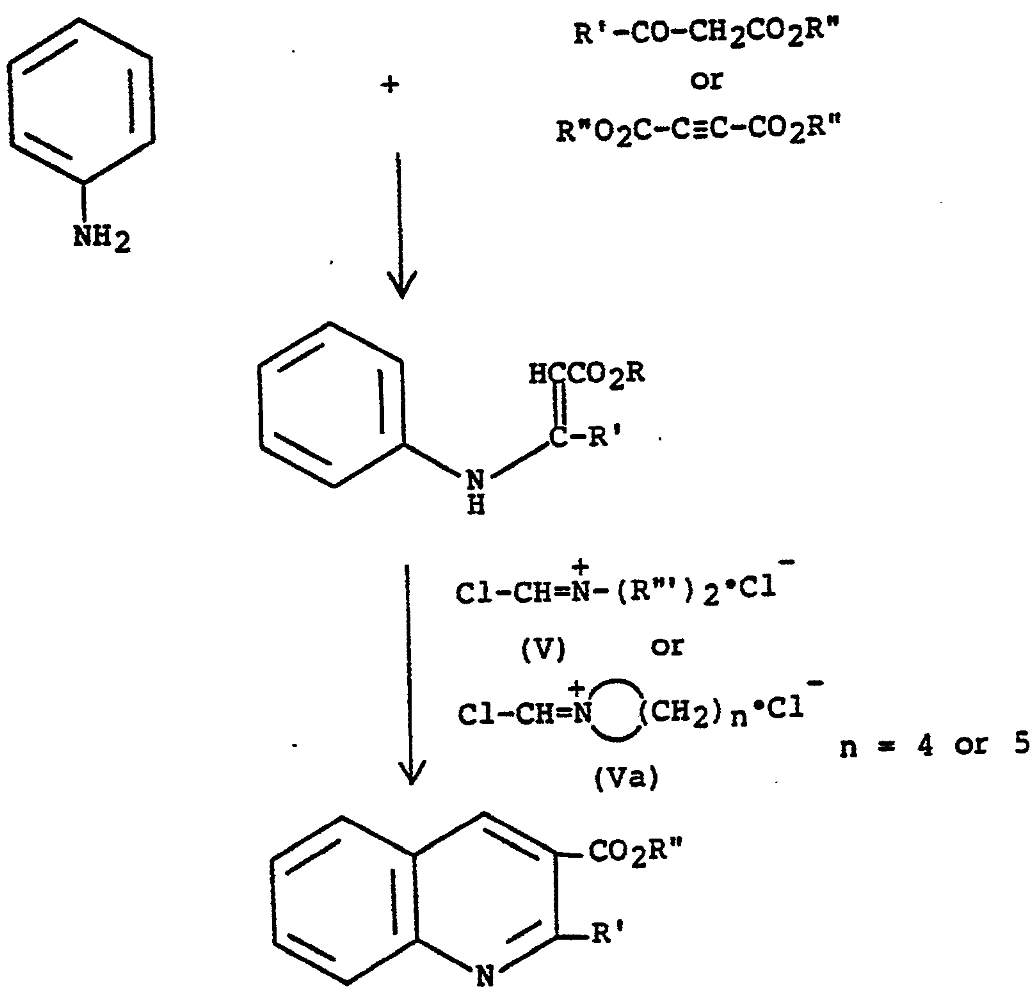


wherein R' is $CH_3$ or $CO_2R''$ and R" is $C_1$-$C_4$ alkyl and R''' is $CH_3$ (or $C_1$-$C_4$ alkyl).

When R' is $CH_3$, the diacid is obtained by concurrent oxidation and hydrolysis of the product under aqueous basic conditions in the presence of nickel peroxide as described in United States Patent 4,459,409.

Unfortunately, the availability of ketoesters and dialkyl acetylene dicarboxylates, such as diethyloxalacetate and diethyl acetylenedicarboxylate, is limited, thus restricting the quantities of anilino-fumarate and quinoline-2,3-dicarboxylic acid, the intermediate required for preparing herbicidal 2-(4-isopropyl-4-methyl-5-oxo-2-imidazolin-2-yl)quinoline-3-carboxylic acid, esters and salts thereof.

SUMMARY OF THE INVENTION

The present invention overcomes this limitation in providing a source of the quinoline-2,3-dicarboxylic acid, esters and salts thereof through the reaction of a dichlorosuccinate and amine to form anilinofumarate. With a ready source of anilinofumarate, an efficient and novel method for a ready source of quinoline-2,3-dicarboxylic acid is available for production of the herbicides, 2-(4-isopropyl-4-methyl-5-oxo-2-imidazolin-2-yl)quinoline-3-carboxylic acid, esters and salts thereof.

It is an object of the present invention, therefore, to provide a novel method for the preparation of anilinofumarate utilizing dichlorosuccinates, obtainable from dialkyl maleates. These are readily available in large quantities and hence provide a method for the manufacture of large quantities of quinoline-2,3-dicarboxylic acid and esters thereof for the subsequent production of herbicidal 2-(4-isopropyl-4-methyl-5-oxo-2-imidazolin-2-yl)quinoline-3-carboxylic acid, esters and salts.

Another object of the present invention is to provide a ready source of anilinofumarates as intermediates for the formation of quinoline-2,3-dicarboxylic acids, precursors of the herbicidal agents, 2-(4-isopropyl-4-methyl-5-oxo-2-imidazolin-2-yl)quinoline-3-carboxylic acid, esters and salts thereof by reacting a dichlorosuccinate with an aniline in an inert organic solvent and aqueous base containing a phase transfer catalyst.

It is another object of the present invention to provide a novel method to prepare dialkyl oxalacetates as precursors for anilinofumarates.

These and further objects will become more apparent by the detailed description of the invention provided hereinbelow.

DETAILED DESCRIPTION OF THE INVENTION

The present invention relates to novel methods for the preparation of anilinofumarate and quinoline-2,3-dicarboxylic acid and esters thereof, said method comprises reacting a dichlorosuccinate (formula I)

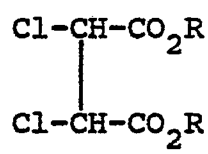

(I)

wherein R is $C_1$-$C_4$ alkyl with a minimum of 3 molar equivalents (3 molars or greater) of an amine having the formula II

$R_1R_2NH$(II)

wherein $R_1$ and $R_2$ are each H or $C_1$-$C_6$ alkyl, with the proviso that only one of $R_1$ or $R_2$ is H; or when taken together $R_1$ and $R_2$ with the nitrogen atom to which they are attached may form a 5 or 6 membered ring containing at most 2 heteroatoms; in an inert solvent, at a temperature of about 25°C to reflux for 1 to 24 hours to form the resulting mixture of alkylaminomaleate or alkylaminofumarate (IIIa),

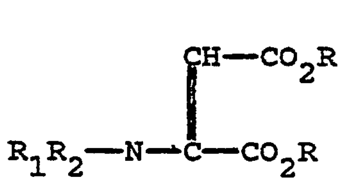

(IIIa)

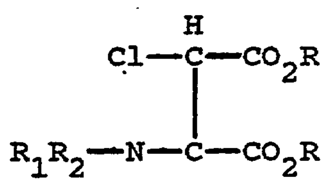

(IIIb)

wherein R, $R_1$ and $R_2$ are as defined above; and further reacting the resulting mixture of alkylaminomaleate or alkylaminofumarate and chloroaminosuccinate of formula IIIa and IIIb with a molar equivalent of aniline in an organic solvent, optionally containing an organic acid, such as acetic acid, at a temperature of 25°C to 90°C for 1 to 24 hours, and isolating the thus-formed anilinofumarate.

Alternatively, the formula (IIIa) alkylaminomaleates or alkylaminofumatates and chloroamino succinates (IIIb) may be hydrolyzed with aqueous acid to yield dialkyl oxalacetates which may then be reacted with aniline.

Quinoline-2,3-dicarboxylate acid then is prepared from the thus-formed anilinofumarate by reaction with an approximately equimolar amount of a Vilsmeier reagent in the presence of a hydrocarbon solvent, such as toluene or a chlorinated hydrocarbon solvent such as methylene chloride, dichloroethane, orthodichlorobenzene, chlorobenzene, or mixtures thereof, at a temperature of about 40°C to 110°C, for a period of time sufficient to essentially complete the reaction and yield a dialkyl quinoline-2,3-dicarboxylate. That is then hydrolyzed under either acid or basic conditions, to yield quinoline-2,3-dicarboxylic acid.

The described reactions are illustrated by Flow Diagrams IIa-c hereinbelow.

## FLOW DIAGRAM IIA

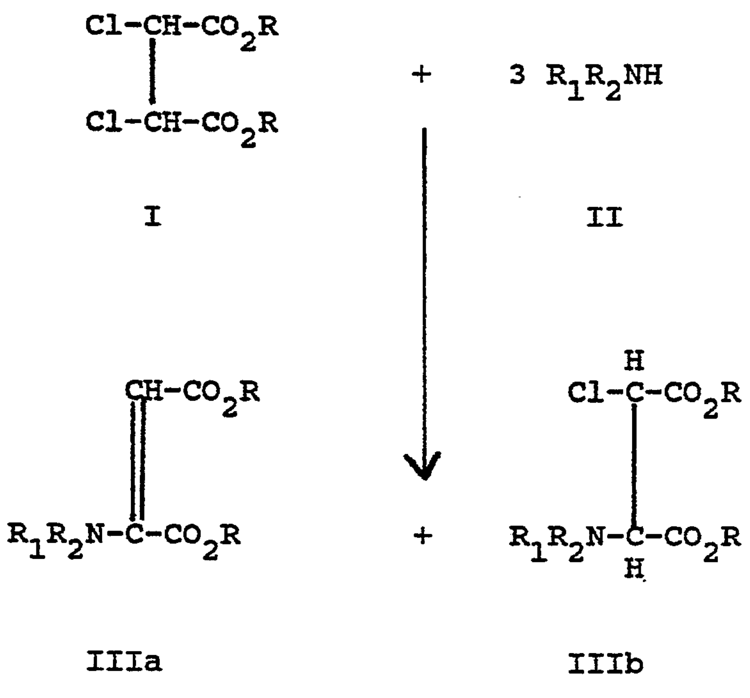

FLOW DIAGRAM IIb

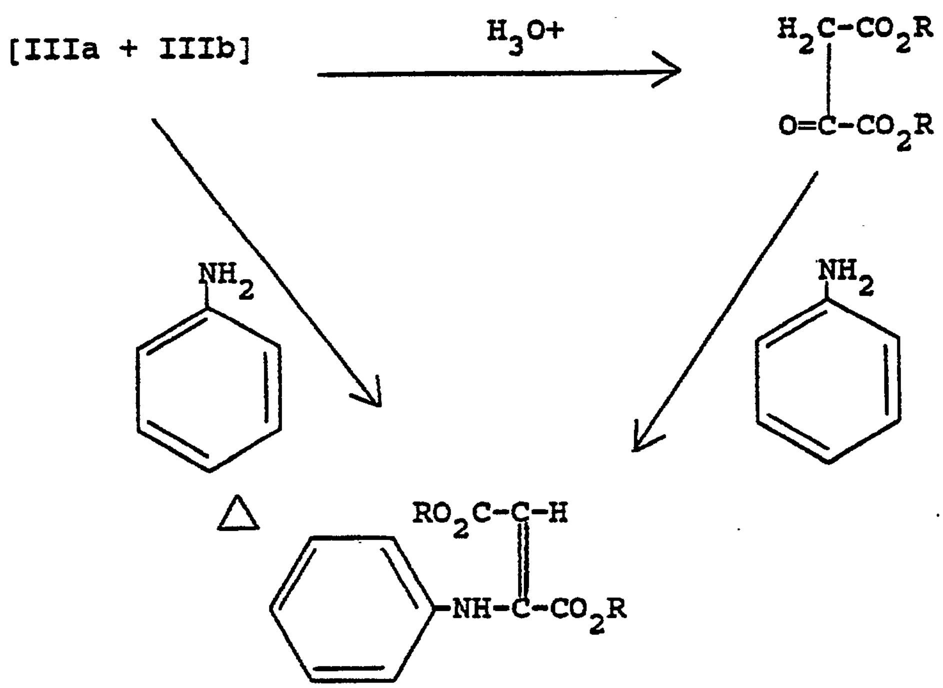

FLOW DIAGRAM IIc

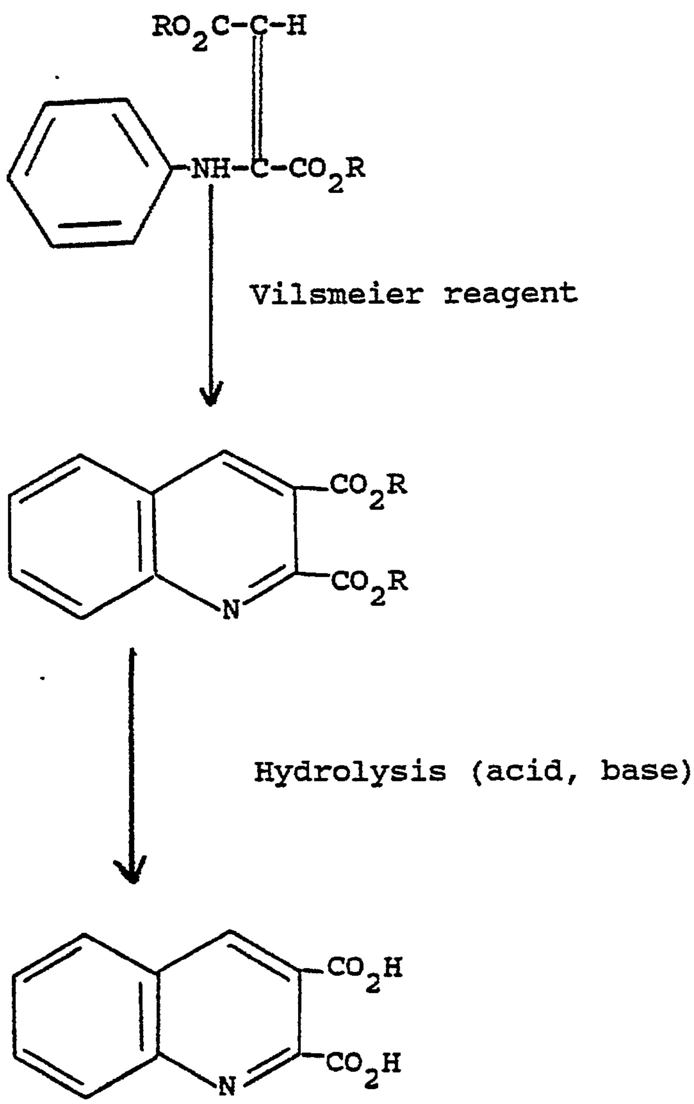


wherein R, $R_1$ and $R_2$ are as described above.

Surprisingly, it has been found that diethyl anilinofumarate or diethyl oxalacetate, and hence quinoline-2,3-dicarboxylic are prepared, in high yields, by the method of the present invention. In accordance with the method of this invention, diethyl dichlorosuccinate, which may be prepared by the method described in Japanese Patent 71 21,564 incorporated herein by reference, is reacted with 3 molar equivalents of diethylamine in toluene at 80°C to 85°C for 7 hours and then at reflux for 3 hours. The reaction mixture is cooled to room temperature, washed with water and the solvent evaporated off to give the formula IIIa and IIIb products (in a ratio of IIIa/IIIb of 7.5/1) wherein $R_1$ and $R_2$ are each ethyl. Solvents suitable for the reaction of dichlorosuccinates with formula II amines include hydrocarbons, aromatic hydrocarbons, halogenated hydrocarbons and aromatic hydrocarbons.

Diethyl anilinofumarate is prepared by adding a molar equivalent of aniline to a toluene solution (20 mL) of the mixture of formula IIIa and IIIb compounds prepared above, containing 5 molar equivalents of acetic acid. The reaction mixture is then stirred at 80°C to 85°C for 4 hours. The product is isolated in 85% to 90% yields after cooling the mixture to room temperature and washing the organic solution with water and then dilute hydrochloric acid and evaporating off the solvent.

Solvents suitable for the reaction of aniline with formula IIIa and IIIb compounds include hydrocarbons, aromatic hydrocarbons and halogenated hydrocarbons and aromatic hydrocarbons. Further, it

has been found that while the above reaction proceeds in the absence of an organic acid, that significantly higher yields of anilinofumarate are obtained in shorter reaction times in the presence of organic acids such as acetic, propionic and the like. Thus, while the above reaction yields 80% to 90% of the desired product after 4 hours at 80°C to 85°C in the presence of acetic acid, refluxing the reactants in the absence of an organic acid for 10 hours yields only 40% anilinofumarate.

The present invention also relates to the following novel methods for preparing anilinofumarates and quinoline-2,3-dicarboxylic acids and esters thereof. These methods comprise reacting a dichlorosuccinate (formula I)

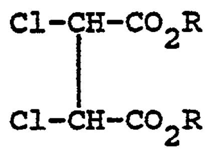

(I)

wherein R is $C_1$-$C_4$ alkyl with a molar equivalent of aniline and a minimum of 2 molar equivalents (equal to or greater than 2 molar equivalents) of aqueous base in the presence of a phase transfer catalyst in an organic solvent at a temperature of 20°C to 90°C for 1 to 24 hours, and isolating the thus-formed anilinofumarate.

Quinoline-2,3dicarboxylate acid is then prepared from the thus-formed anilinofumarate by reacting the anilinofumarate with an approximately equi-molar amount of a Vilsmeier reagent (immonium salt) in the presence of a hydrocarbon solvent, such as toluene, or a chlorinated hydrocarbon solvent such as methylene chloride, dichloroethane, ortho dichlorobenzene, chlorobenzene, or mixtures thereof, at a temperature of about 40°C and 130°C, for a period of time sufficient to essentially complete the reaction and yield a dialkyl quinoline-2,3-dicarboxylate. This quinoline-2,3-dicarboxylate is hydrolyzed, under either acid or basic conditions, to give quinoline-2,3-dicarboxylic acid.

The above reactions are graphically illustrated in Flow Diagrams IIa-b.

## FLOW DIAGRAM IIIA

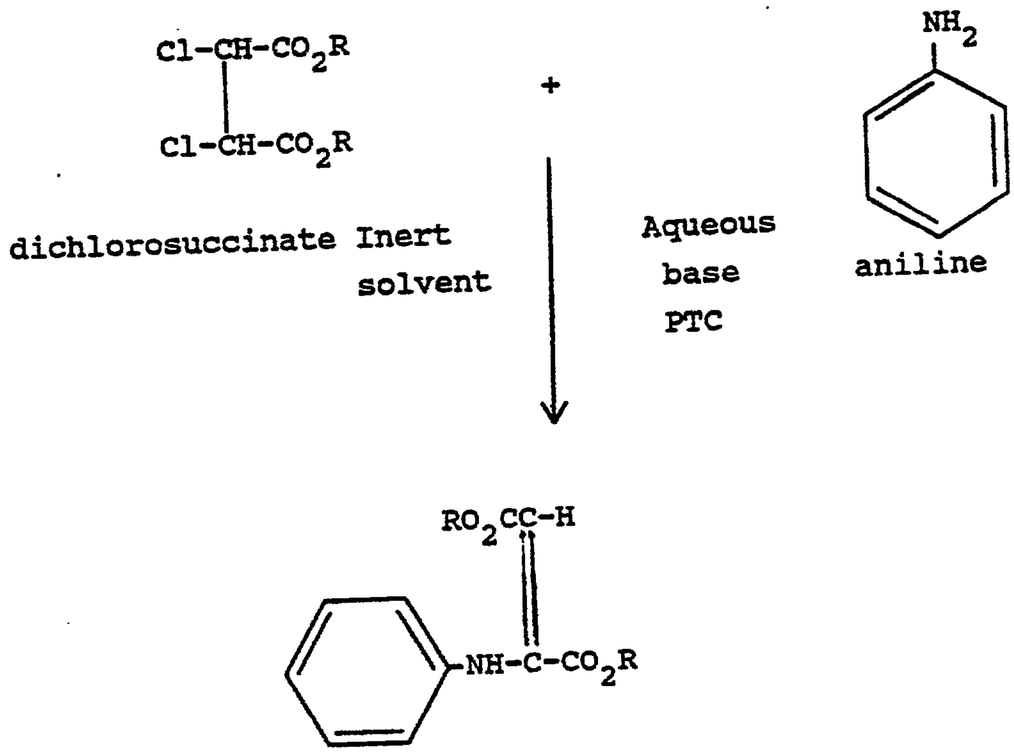


dichlorosuccinate

aniline

anilinofumarate

## FLOW DIAGRAM IIIB

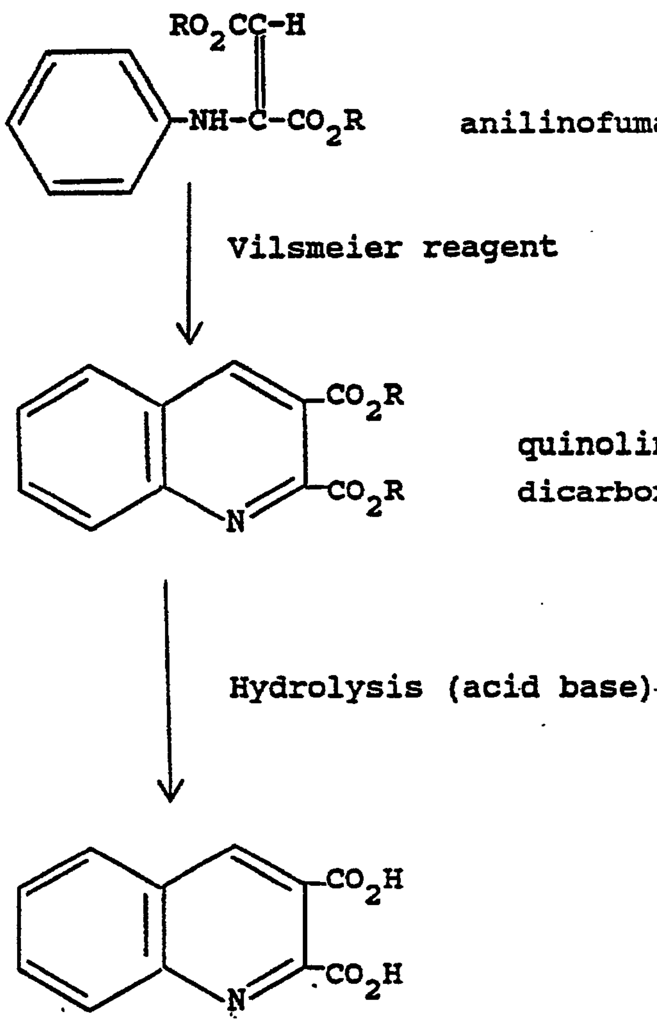


anilinofumarate

quinoline-2,3-dicarboxylate

quinoline-2,3-dicarboxylic acid

wherein R is as described in formula I.

Surprisingly, it has been found that aniline may be reacted with dialkyl dichlorosuccinates by the method of this invention to directly yield anilinofumarate. This procedure eliminates a processing step required in the recently-discovered novel synthesis of anilinofumarate that first reacts dialkyl dichlorosuccinate with a primary or secondary amine and then reacts the resulting product mixture with aniline.

The novel method of the present invention thus provides a simple and more direct method for the production of anilinofumarate. This method, in turn, reduces handling processing, effluent and exposure to intermediate reactions and reagents, resulting in a process that is cleaner, safer, cheaper and more efficient than prior reported methods.

In accordance with the method of this invention, diethyl dichlorosuccinate (0.01 mol), in monochlorobenzene is reacted with aniline (0.01 mol) by stirring the mixture in the presence of 0.3 molar equivalents of aqueous sodium hydroxide (33% NaOH w/w) and a catalytic amount (5 mol %) of tetrabutylammonium chloride at 75°C-80°C for 2 hours and 30 minutes. The thus-formed anilinofumarate is readily isolated by adding water, washing the organic layer, spearating off the organic phase and removing the solvent.

Aqueous bases suitable for the preparation of anilinofumarate by the present method include sodium and potassium hydroxide, carbonate and bicarbonate at concentrations of 15% to 50% by weight, in amounts sufficient to provide about 2 molar equivalents of available base, hence the above identified bases are preferrably employed in from 1-3 molar equivalents. The carbonates are employed in 1 to 3 molar equivalents and the hydroxides and bicarbonates employed at 2 to 3 molar equivalents.

Quaternary ammonium phase transfer catalysts, such as tetrabutylammonium chloride and benzyltriethylammonium chloride, in amounts as little as 0.05 molar equivalents, have demonstrated comparable results for the method of this invention. Other reagents capable of catalyzing two phase reactions and exhibiting stability under the conditions of this reaction are equally suitable.

Surprisingly it has been found that the use of 1.1 to 3.0 molar equivalents of 15% to 50% aqueous sodium carbonate or potassium carbonate, preferably in conjunction with the use of 2 to 10 mol % of tricaprylylmethylammonium chloride or tributylmethylammonium chloride results in unexpectly high yields of anilinofumarate.

Generally, the organic solvents which find utility in the method of the present invention are inert to the reaction conditiond and include such colvents as hydrocarbons, aromatic hydrocarbons and chlorinated derivatives thereof, with chlorinated aromatic hydrocarbons, such as chlorobenzene, and aromatic solvents, such as toluene, being preferred.

The reaction of the invention proceeds at varying rates at a temperature range of 20°C to 90°C, with 20°C to 85°C being preferred, 20°C to 80°C most preferred.

The methods of the present invention are further exemplified by the following examples which are illustrative and not limitative thereof.

EXAMPLE 1

Preparation of dichlorosuccinate

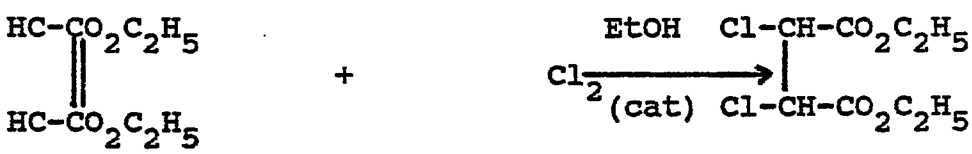


Chlorine gas is bubbled into an ethylene dichloride solution of diethyl maleate containing ethanol, (0.1 molar equivalents). After stirring the mixture at room temperature for 8 hours, it is flushed with nitrogen gas for 5 minutes and the solvent removed under reduced pressure to yield the dichlorosuccinate in 94% yield.

EXAMPLE 2-14

Preparation of diethyl anilinofumarate

EXAMPLE 2-14

Preparation of diethyl anilinofumarate

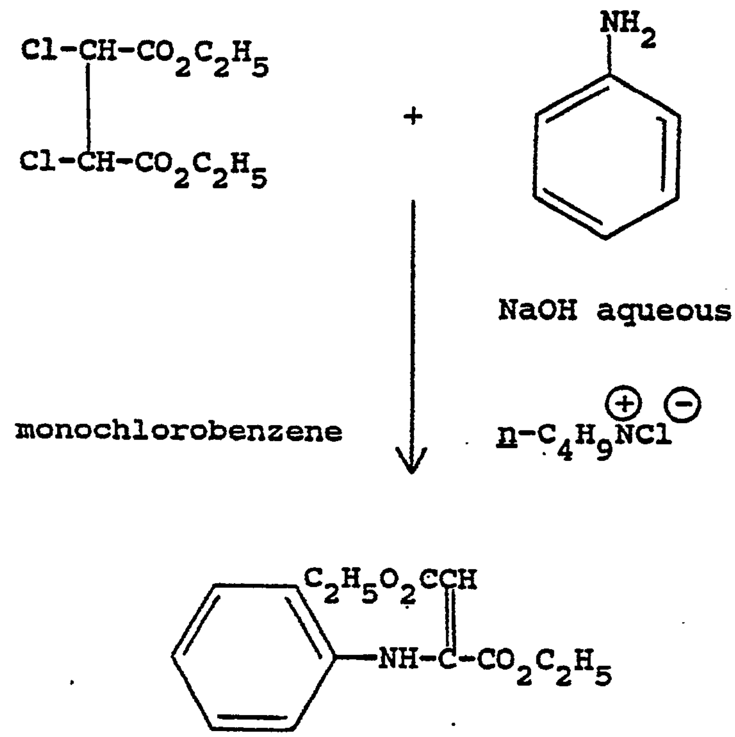


Anilin (0.93) g, 0.01 mol) is added to as stirred solution of diethyl dichlorosuccinate (prepared as described in example 1 of the above from diethyl maleate (0.01 mol) in monochlorobenzene). Aqueous sodium hydroxide (3.6 g, 33% w/w. 0.03 mol) and tetrabutylammonium chloride (0.14 g, 0.005 mol) are then added, at room temperature. The resulting mixture is stirred at 75°C to 80°C for 2 hours and 30 minutes. The mixture then is cooled to room temperature, and water (10 mL) is added to the stirred mixture. The aqueous layer is separated, and the resulting organic solution analyzed for anilinofumarate by gas liquid chromatography. Isolation of the product by removal of the solvent yields 1.25 g of anilinofumarate.

Utilizing the above procedure with various phase transfer catalysts, aqueous bases of varying concentrations and solvents gives anilinofumarate as illustrated in Table II.

Table I

| Example | Phase transfer Catalyst/(mol %) | Solvent | Temp. °C | Time | Base (conc) | Base mols | % Yield anilino-fumarate (based on starting diethyl maleate) |
|---|---|---|---|---|---|---|---|
| 3 | Benzyltriethyl-ammonium chloride (5.0) | monochloro-benzene | 75 - 80 | 2.0 | NaOH (33%)<br>$K_2CO_3$ | 0.2<br>0.1 | 32 |
| 4 | Tetrabutyl-ammonium chloride (5.0) | monochloro-benzene | 75 - 80 | 2.5<br>3.0 | NaOH (33%) | 0.3 | 48 |
| 5 | Benzyltriethyl-ammonium chloride (5.0) | monochloro-benzene | 30 - 43 then 25 - 30 | 0.5<br>3.0 | NaOH (33%) | 0.3 | 32 |
| 6 | Benzyltriethyl-ammonium chloride (5.0) | monochloro-benzene | 25 - 48 then 25 - 30 | 0.5<br>2.5 | NaOH (50%) | 0.3 | 18 |

Table I (Cont.)

| Example | Phase transfer Catalyst/(mol %) | Solvent | Temp. °C | Time hr. | Base (conc) | Base mols | % Yield anilino-fumarate (based on starting diethyl maleate) |
|---|---|---|---|---|---|---|---|
| 7 | Tricaprylylmethyl-ammonium chloride (5.0) | toluene | 75-80 | 10 | $Na_2CO_3$ (20%) | 2.2 | 78 |
| 8 | Tricaprylylmethyl-ammonium chloride (5.0) | toluene | 75-80 | 6 | $Na_2CO_3$ (40%) | 2.2 | 43 |
| 9 | Tributylmethyl-ammonium chloride (6.0) | toluene | 75-80 | 10 | $Na_2CO_3$ (20%) | 2.2 | 56 |
| 10 | Tricaprylylmethyl-ammonium chloride (5.0) | toluene | 75-80 | 10 | $NaHCO_3$ (15%) | 2.5 | 66 |
| 11 | Tricaprylylmethyl-ammonium chloride (5.0) | toluene | 75-80 | 5 | $K_2CO_3$ (50%) | 3.0 | 62 |

Table I (Cont.)

| Example | Phase transfer Catalyst/(mol %) | Solvent | Temp. °C | Time hr. | Base (conc) | Base mols | % Yield anilino-fumarate (based on starting diethyl maleate) |
|---|---|---|---|---|---|---|---|
| 12 | Tricaprylylmethyl-ammonium chloride (5.0) | toluene | 75-80 | 10 | $Na_2CO_3$ (20%) | 1.2 | 73 |
| 13 | Benzyltriethyl-ammonium chloride (6.0) | toluene | 75-80 | 10 | $Na_2CO_3$ (20%) | 2.2 | 8 |
| 14 | None | toluene | 75-80 | 10 | $Na_2CO_3$ (20%) | 2.2 | 1 |

EXAMPLE 15-18

Preparation of diethyl diethylaminomaleate and diethyl 2-chloro-3-diethylaminosuccinate

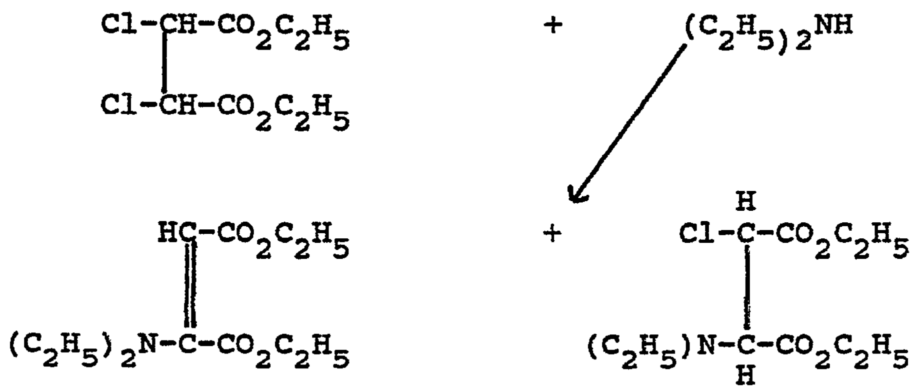

Diethylamine (2.41 g, 0.033 mol) is added dropwise to stirred solution of diethyl dichlorosuccinate (2.59 g, 0.01 mol) in toluene (15 mL). The resulting mixture is heated at 80°C to 85°C for 8 hours and then at reflux for 3 hours. After cooling the reaction mixture to room temperature, it is washed with water (15 mL) and the toluene layer is separated off and evaporated under reduced pressure to yield 2.07 g (85%) of the mixture of diethyl diethylaminomaleate and the title chloro-amino succinate in a maleate to succinate ratio of 7.5/1.

Utilizing the above procedure and substituting the appropriate amine for diethylamine yields the products listed in Table II.

TABLE II

| Example | Amine | % Yield (maleate & succinate) | Maleate to succinate Ratio |
|---|---|---|---|
| 3 | 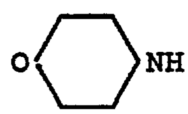 | 86 | 4:1 |
| 4 | $HNC_4H_9\underline{n}$ | 44 (fumarate) | - |
| 5 | 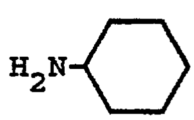 | 52 (fumarate) | - |

EXAMPLE 19-22

Preparation of anilinofumarate

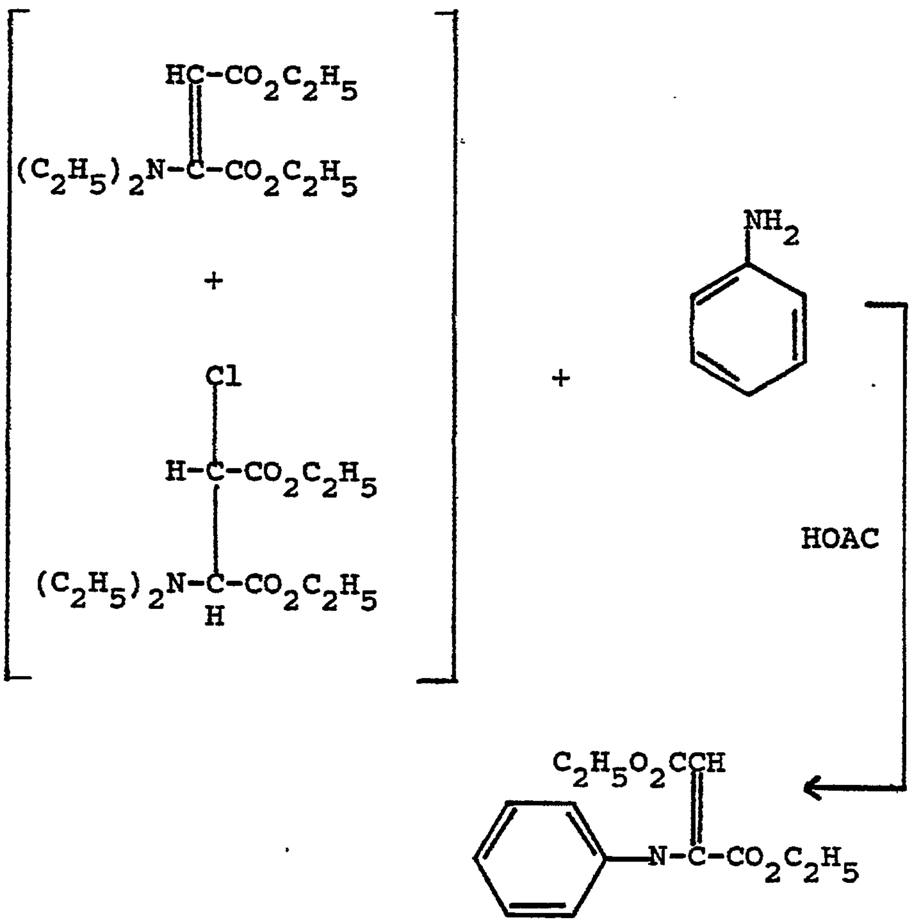

Aniline (0.93 g, 0.01 mol) is added to a toluene (20 mL) solution of acetic acid (3.0 g, 0.05 mol) and the mixture of diethyl diethylaminomaleate and diethyl 2-chloro-3-diethylaminosuccinate prepared in Example 2 above. The resulting solution is heated at 80°C to 85°C for 4 hours. After cooling the reaction mixture to room temperature, it is washed with water (10 mL) and then with aqueous HCl (12% w/w, 2 mL).

Analysis of the resulting toluene solution by gas liquid chromatography and isolation of the product indicate an overall yield of the sequence starting from diethylmaleate to be 69%.

Utilizing the above procedure the alkylaminomaleate and chloroalkylaminosuccinate mixtures obtained in Examples 2-5 give the yields of anilinofumarate based on starting diethylmaleate listed in Table III.

TABLE III

| Example | Starting material of example | | % Yield for sequence starting from diethylmaleate or diethylfumarate |
|---|---|---|---|
| 7 | 3 | | 67% |
| 8 | 4 | | 38% |
| 9 | 5 | | 45% |
| 10 | 2 | no organic acid reflux for 10 hours | 40% |

EXAMPLE 23

Preparation of diethyloxalacetate and subsequent preparation of diethylanilinofumarate

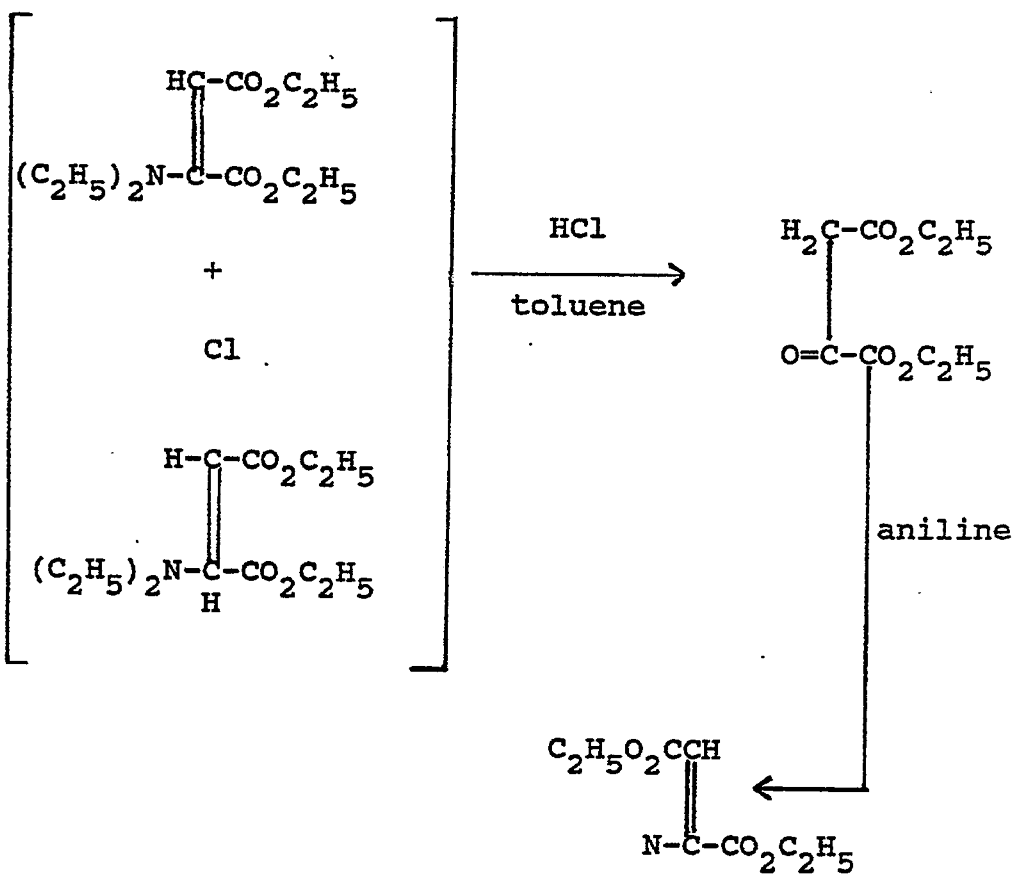

A toluene (15 mL) solution of a mixture of diethyl diethylaminomaleate and diethyl 2-chloro-3-diethylaminosuccinate (2.43 g, 0.01 mol) is prepared by the procedure of Example 2 above and is stirred with water (5.0 g) containing 2.15 g (0.015 mol) of concentrated HCl for two (2) hours and 30 minutes. The toluene layer containing diethyl oxalacetate is separated off and aniline (0.93 g, 0.01 mol) added. The resulting solution is stirred at room temperature for 30 minutes and then at reflux for one hour and 30 minutes while collecting the water which is formed in a Dean Stark collector. Analysis of the cooled toluene solution by gas liquid chromatography shows the yield of diethylanilinofumarate to be 55%.

Example 24

Preparation of quinoline-2,3-dicarboxylic acid

Vilsmeier reagent is prepared by adding 4.61 g (0.03 mol) of $POCl_3$, dropwise, to a solution of 2.19 g (0.03 mol) of dimethylformamide in 12 mL of toluene, while maintaining the temperature at 20°C to 30°C. The two layers are stirred at 20°C to 30°C for 60 minutes and then treated, dropwise, with a solution of 5.26 g (0.02 mol) of diethyl anilinofumarate prepared by the procedure of example 2 above, in 40 mL of toluene, while maintaining the temperature at 20°C to 30°C The solution that forms on heating is refluxed for 2 hours, cooled until reflux stops and is poured into 60 mL of water. The dark syrupy material that precipitates dissolves, when stirred at room temperature for 30 minutes. Analysis of the toluene solution by glc indicates a yield of 72%. Evaporation of the diester solution gives an oily low melting solid, which upon recrystallization from isopropyl alcohol gives 4.05 g of tan solid, mp 53-56°C.

Two phases that are formed from 4.1 g (0.015 mol) of diester in 25 mL of toluene and 16 mL of 15% NaOH are refluxed with good mixing for 8 hours. The two phases are cooled to 50°C to 55°C and diluted with 20 mL of water. The aqueous phase is separated and added dropwise to 11 mL of 35% $H_2SO_4$, while keeping the temperature less than 40°C, and the resulting thick mixture is filtered, and the solid collected and dried overnight at 60°C/30-50 mmHg to yield 3.19 g of quinoline-2,3-dicarboxylic acid.

**Claims**

1. A method for the preparation of anilinofumarate, said method comprising: reacting a dichlorosuccinate of formula I

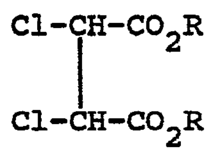

(I)

wherein R is $C_1$-$C_4$ alkyl, with a minimum of 3 molar equivalents of an amine of formula II

$R_1R_2NH$ (II)

wherein $R_1$ and $R_2$ are each H or $C_1$-$C_6$ alkyl, with the proviso that only one of $R_1$ or $R_2$ is H; or when taken together $R_1$ and $R_2$ with the nitrogen atom to which they are attached form a 5 or 6 membered ring containing at most 2 heteroatoms; in an inert solvent at a temperature of about 25°C to reflux for 1 to 24 hours; and further reacting the resulting mixture of formula IIIa alkylaminomaleate or alkylaminofumarate and formula IIIb chloroaminosuccinate

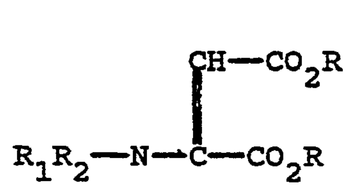

(IIIa)

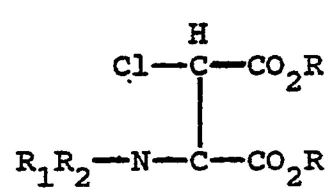

(IIIb)

wherein R, $R_1$ and $R_2$ are as described hereinabove for formula I and formula II with a molar equivalent of aniline in an inert organic solvent containing an organic acid at a temperature of 25°C to 90°C for 1 to 24 hours.

2. A method according to Claim 1, wherein said organic acid is acetic acid; wherein R is $C_2H_5$; wherein said formula II amine is diethylamine or morpholine; and wherein said solvent is chlorinated hydrocarbon, chlorinated aromatic hydrocarbon, aromatic hydrocarbon, or mixtures thereof.

3. A method according to Claim 2, wherein said solvent is ethylenedichloride, monochlobenzene, toluene or mixtures thereof.

4. A method according to Claim 3, wherein said reactions are conducted at a temperature of 75°C to 85°C for 2 to 9 hours.

5. A method for the preparation of quinoline-2,3-dicarboxylic acid, said method comprising: reacting a dichlorosuccinate of formula I

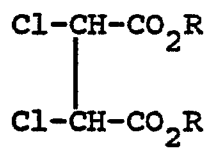

I

wherein R is $C_1$-$C_4$ alkyl, with a minimum of 3 molar equivalents of an amine of formula II

$R_1R_2NH$ (II)

wherein $R_1$ and $R_2$ are each H or $C_1$-$C_6$ alkyl, with the proviso that only one of $R_1$ or $R_2$ is H; or when taken together $R_1$ and $R_2$ with the nitrogen atom to which they are attached form a 5 or 6 membered ring containing at most 2 heteroatoms; in an inert solvent at a temperature of about 25°C to reflux for 1 to 24 hours; and further reacting the resulting mixture of formula IIIa alkylaminomaleate or alkylaminofumarate and formula IIIb chloroaminosuccinate

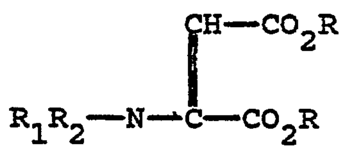

(IIIa)

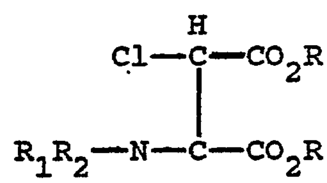

(IIIb)

wherein R, $R_1$ and $R_2$ are as described hereinabove for formula I and formula II with a molar equivalent of aniline in an inert organic solvent containing an organic acid; at a temperature of 25°C to 90°C for 1 to 24 hours; reacting the thus-formed anilinofumarate with an equimolar amount of a Vilsmeier reagent in the presence of a hydrocarbon solvent or a chlorinated hydrocarbon solvent, at a temperature of 40°C to 110°C, for a period of time sufficient to essentially complete the reaction and yield a dialkyl quinoline-2,3-dicarboxylate; hydrolyzing said dialkyl quinoline-2,3-dicarboxylate to quinoline-2,3-dicarboxylic acid.

6. A method according to Claim 5, wherein said organic acid is acetic acid; wherein R is $C_2H_5$; wherein said formula II amine is diethylamine or morpholine; and wherein said inert organic solvent is chlorinated hydrocarbon, chlorinated aromatic hydrocarbon, aromatic hydrocarbon, or mixtures thereof.

7. A method according to Claim 6, wherein said inert organic solvent is ethylenedichloride, monochlobenzene, toluene, or mixtures thereof; wherein said Vilsmeier reagent solvent is a hydrocarbon, chlorinated hydrocarbon, or mixtures thereof; and wherein said solvent is toluene, methylene chloride, dichloroethane, ortho dichlorobenzene, chlorobenzene, or mixtures thereof.

8. A method for the preparation of dialkyloxalacetates having the structure

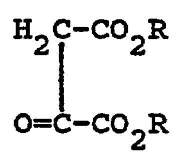

said method comprising: reacting a dichlorosuccinate of formula I

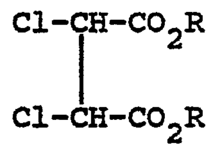

(I)

wherein R is $C_1$-$C_4$ alkyl, with a minimum of 3 molar equivalents of an amine of formula II

$R_1R_2NH$

wherein $R_1$ and $R_2$ are each H or $C_1$-$C_6$ alkyl, with the proviso that only one of $R_1$ or $R_2$ is H; or when taken together $R_1$ and $R_2$ with the nitrogen atom to which they are attached form a 5 or 6 membered ring containing at most 2 heteroatoms; in an inert solvent at a temperature of about 25°C to reflux for one (1) to 24 hours; and further reacting the resulting mixture of formula IIIa alkylaminomaleate or alkylaminofumarate and formula IIIb chloroaminosuccinate

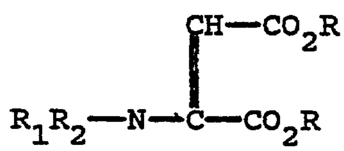

(IIIa)

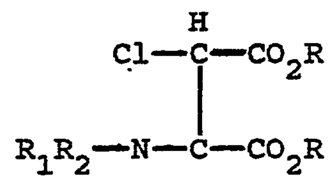

(IIIb)

wherein R, $R_1$ and $R_2$ are as hereinabove described for formula I and formula II, with aqueous acid.

9. A method for the preparation of anilinofumarate, said method comprising: reacting a dichlorosuccinate of formula I

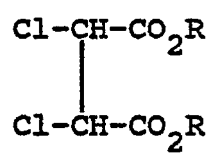

(I)

wherein R is $C_1$-$C_4$ alkyl, with a molar equivalent of aniline in an inert organic solvent and 2 or greater molar equivalents of an aqueous base in the presence of a phase transfer catalyst at a temperature of 20°C to 90°C for 1 to 24 hours.

10. A method according to Claim 9, wherein said aqueous base is 15% to 50%, on a weight basis, of sodium hydroxide, potassium hydroxide or mixtures thereof; wherein said phase transfer catalyst is a quaternary ammonium salt consisting of such salts as n-butylammonium chloride, benzyltriethylammonium chloride, or mixtures thereof; wherein said organic solvent is a hydrocarbon, aromatic hydrocarbon, chlorinated aromatic hydrocarbon, or mixtures thereof; wherein said solvent is a chlorinated aromatic hydrocarbon, aromatic hydrocarbon, or mixtures thereof; wherein said base is 20% to 50%, on a weight basis, of sodium hydroxide, potassium hydroxide, or mixtures thereof; wherein said base is 2 to 3 molar equivalents; and wherein said temperature is 20°C to 85°C.

11. A method according to Claim 10, wherein said aqueous base is 1.1 to 3.0 molar equivalents of 15% to 50%, on a weight basis, of sodium carbonate, potassium carbonate or a mixture thereof; and wherein said quaternary ammonium salt is tricaprylylmethylammonium chloride or tributyl,ethylammonium chloride.

## Revendications

1. Procédé pour la préparation d'anilinofumarate, ledit procédé comprenant la mise en réaction d'un dichlorosuccinate de formule I

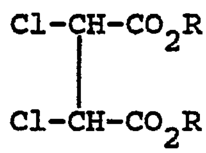

(I)

dans laquelle R est un groupe alkyle en $C_1$–$C_4$, avec un minimum de 3 équivalents molaires d'une amine de formule II

$R_1R_2NH$ (II)

dans laquelle $R_1$ et $R_2$ sont chacun H ou un groupe alkyle en $C_1$–$C_6$, étant entendu qu'un seul des radicaux $R_1$ et $R_2$ est H; ou $R_1$ et $R_2$ peuvent former ensemble, avec l'atome d'azote auquel ils sont liés, un cycle à 5 ou 6 chaînons contenant au maximum deux hétéroatomes; dans un solvant inerte, à une température dans la plage allant d'environ 25°C à la température de reflux, pendant 1 à 24 heures, et la mise en réaction à son tour du mélange résultant d'alkylaminomaléate ou alkylaminofumarate de formule IIIa et de chloroaminosuccinate de formule IIIb

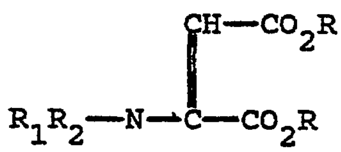

(IIIa)

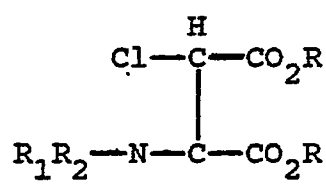

(IIIb)

R, $R_1$, et $R_2$ étant tels que définis ci-dessus pour la formule I et la formule II, avec 1 équivalent molaire d'aniline dans un solvant organique inerte contenant un acide organique, à une température de 25°C à 90°C, pendant 1 à 24 heures.

2. Procédé selon la revendication 1, dans lequel ledit acide organique est l'acide acétique; dans lequel R est $C_2H_5$; dans lequel l'amine de formule II est la diéthylamine ou la morpholine; et dans lequel ledit solvant est un hydrocarbure chloré, un hydrocarbure aromatique chloré, un hydrocarbure aromatique ou des mélanges de ceux-ci.

3. Procédé selon la revendication 2, dans lequel ledit solvant est le dichlorure d'éthylène, le monochlorobenzène, le toluène ou des mélanges de ceux-ci.

4. Procédé selon la revendication 3, dans lequel lesdites réactions sont effectuées à une température de 75 à 85°C pendant 2 à 9 heures.

5. Procédé pour la préparation de l'acide quinoléine-2,3-dicarboxylite, ledit procédé comprenant la mise en réaction d'un dichlorosuccinate de formule I

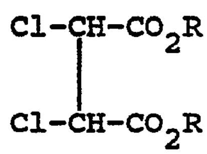

(I)

dans laquelle R est un groupe alkyle en $C_1$–$C_4$, avec un minimum de 3 équivalents molaires d'une amine de formule II

$R_1R_2NH$ (II)

dans laquelle $R_1$ et $R_2$ sont chacun H ou un groupe alkyle en $C_1$–$C_6$, étant entendu qu'un seul des radicaux $R_1$ et $R_2$ est H; ou $R_1$ et $R_2$ peuvent former ensemble, avec l'atome d'azote auquel ils sont liés, un cycle à 5 ou 6 chaînons contenant au maximum deux hétéroatomes; dans un solvant inerte, à une température dans la plage allant d'environ 25°C à la température de reflux, pendant 1 à 24 heures, et la mise en réaction à son tour du mélange résultant d'alkylaminomaléate ou alkylaminofumarate de formule IIIa et de chloroaminosuccinate de formule IIIb

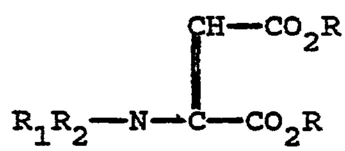

(IIIa)

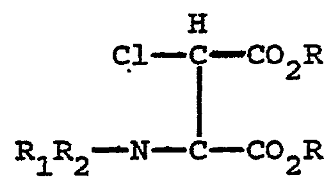

(IIIb)

R, $R_1$ et $R_2$ étant tels que définis ci-dessus pour la formule I et la formule II, avec un équivalent molaire d'aniline dans un solvant organique inerte contenant un acide organique, à une température de 25 à 90°C, pendant 1 à 24 heures; la mise en réaction de l'anilinofumarate ainsi formé avec un équivalent molaire d'un réactif de Vilsmeier en présence d'un solvant de type hydrocarbure ou d'un solvant de type hydrocarbure chloré, à une température de 40 à 110°C, pendant une durée suffisante pour achever pratiquement la réaction et obtenir un quinoléine-2,3-dicarboxylate de dialkyle; l'hydrolyse dudit quinoléine-2, 3-dicarboxylate de dialkyle en acide quinoléine-2,3-dicarboxylique.

6. Procédé selon la revendication 5, dans lequel ledit acide organique est l'acide acétique; dans lequel R est $C_2H_5$; dans lequel l'amine de formule II est la diéthylamine ou la morpholine; et dans lequel ledit solvant organique inerte est un hydrocarbure chloré, un hydrocarbure aromatique chloré, un hydrocarbure aromatique ou des mélange de ceux-ci.

7. Procédé selon la revendication 6, dans lequel ledit solvant organique inerte est le dichlorure d'éthylène, le monochlorobenzène, le toluène, ou des mélanges de ceux-ci; dans lequel ledit solvant pour le réactif de Vilsmeier est un hydrocarbure, un hydrocarbure chloré ou des mélanges de ceux-ci; et dans lequel ledit solvant est le toluène, le chlorure de méthylène, le dichloréthane, l'o-dichlorobenzène, le chlorobenzène, ou des mélanges de ceux-ci.

8. Procédé pour la préparation d'oxaloacétates de dialkyle de formule

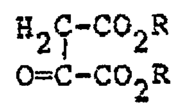

ledit procédé comprenant la mise en réaction d'un dichlorosuccinate de formule I

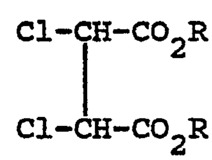

(I)

dans laquelle R est un groupe alkyle en C1–C4, avec un minimum de 3 équivalents molaires d'une amine de formule II

$R_1R_2NH$

dans laquelle $R_1$ et $R_2$ sont chacun H ou un groupe alkyle en $C_1$–$C_6$, étant entendu qu'un seul des radicaux $R_1$ et $R_2$ est H; ou $R^1$ et R2 forment ensemble, avec l'atome d'azote auquel ils sont liés, un cycle à 5 ou 6 chaînons contenant au maximum deux hétéroatomes; dans un solvant inerte, à une température dans la plage allant d'environ 25°C à la température de reflux, pendant 1 à 24 heures, et la mise en réaction à son tour du mélange résultant d'alkylaminomaléate ou alkylaminofumarate de formule IIIa et de chloroaminosuccinate de formule IIIb

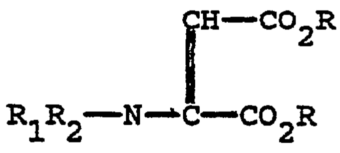

(IIIa)

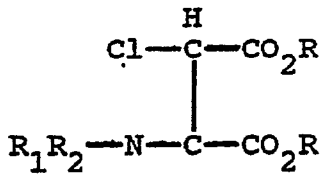

(IIIb)

R, $R_1$ et $R_2$ étant tels que définis ci-dessus pour la formule I et la formule II, avec un acide en solution aqueuse.

9. Procédé pour la préparation d'anilinofumarate, ledit procédé comprenant la mise en réaction d'un dichlorosuccinate de formule I

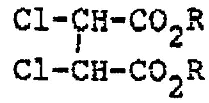

I

dans laquelle R est un groupe alkyle en $C_1$–$C_4$, avec un équivalent molaire d'aniline dans un solvant organique inerte et 2 équivalents molaires ou plus d'une base en solution aqueuse en présence d'un catalyseur de transfert de phase, à une température de 20 à 90°C, pendant 1 à 24 heures.

10. Procédé selon la revendication 9, dans lequel ladite base aqueuse est à 15–50% en poids d'hydroxyde de sodium, hydroxyde de potassium ou des mélanges de ceux-ci; dans lequel ledit catalyseur de transfert de phase est un sel d'ammonium quaternaire constitué de sels tels que le chlorure de n-butylammonium, le chlorure de benzyltriéthylammonium, ou des mélanges de ceux-ci; dans lequel ledit solvant organique est un hydrocarbure, un hydrocarbure aromatique ou un hydrocarbure aromatique chloré, ou des mélanges de ceux-ci; dans lequel ledit solvant est un hydrocarbure aromatique chloré, un hydrocarbure aromatique, ou des mélanges de ceux-ci; dans lequel ladite base est à 20–50%, en poids, d'hydroxyde de sodium, d'hydroxyde de potassium ou des mélanges de ceux-ci; dans lequel ladite base représente 2 à 3 équivalents molaires; et dans lequel ladite température est de 20 à 85°C.

11. Procédé selon la revendication 10, dans lequel ladite base en solution aqueuse est de 1,1 à 3,0 équivalents molaires de 15 à 50%, en poids, de carbonate de sodium, carbonate de potassium ou d'un mélange de ceux-ci; et dans lequel ledit sel d'ammonium quaternaire est le chlorure de tricaprylylméthylammonium ou le chlorure de tributyléthylammonium.

**Patentansprüche**

1. Verfahren zur Herstellung von Anilinofumarat, umfassend die Umsetzung eines Dichlorsuccinats der Formel I

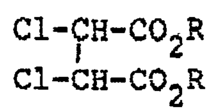

I

wobei R für $C_{1-4}$-Alkyl steht, mit einem Minimum von drei molaren Aquivalenten eines Amins der Formel II

$R_1R_2NH$ (II)

wobei $R_1$ und $R_2$ jeweils für H oder $C_{1-6}$-Alkyl stehen, mit der Maßgabe, daß nur einer von $R_1$ oder $R_2$ H ist, oder $R_1$ und $R_2$ zusammengefaßt mit dem Stickstoffatom, an das sie geknüpft sind, einen 5- oder 6-gliedrigen Ring bilden, der höchstens zwei Heteroatome enthält; in einem inerten Lösungsmittel, bei einer Temperatur von etwa 25°C bis zum Rückfluß, während 1 bis 24 Stunden; und weitere Umsetzung der resultierenden Mischung von Alkylaminomaleat oder Alkylaminofumarat der Formel IIIa und Chloraminosuccinat der Formel IIIb

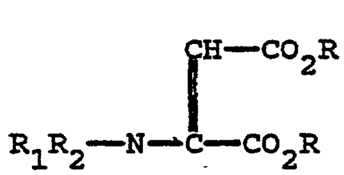

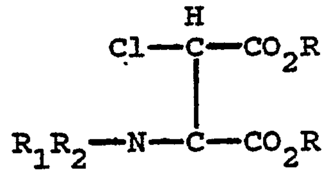

(IIIa) (IIIb)

wobei R, $R_1$ und $R_2$ wie vorstehend für Formel I und Formel II beschrieben sind, mit einem molaren Äquivalent Anilin in einem inerten,· organischen Lösungsmittel, das eine organische Säure enthält, bei einer Temperatur von 25°C bis 90°C während 1 bis 24 Stunden.

2. Verfahren gemäß Anspruch 1, wobei die organische Säure Essigsäure ist; wobei R für $C_2H_5$ steht; wobei das Amin der Formel II Diethylamin oder Morpholin ist und wobei es sich bei dem Lösungsmittel um chlorierten Kohlenwasserstoff, chlorierten, aromatischen Kohlenwasserstoff, aromatischen Kohlenwasserstoff oder Mischungen derselben handelt.

3. Verfahren gemäß Anspruch 2, wobei es sich bei dem Lösungsmittel um Ethylendichlorid, Monochlorbenzol, Toluol oder Mischungen derselben handelt.

4. Verfahren gemäß Anspruch 3, wobei die Reaktionen bei einer Temperatur von 75°C bis 85°C, während 2 bis 9 Stunden durchgeführt werden.

5. Verfahren zur Herstellung von Chinolin-2,3-dicarbonsäure, umfassend die Umsetzung eines Dichlorsuccinats der Formel I

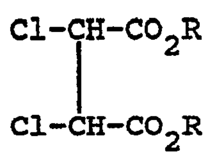

(I)

wobei R für $C_{1-4}$-Alkyl steht mit einem Minimum von 3 molaren Äquivalenten eines Amins der Formel II

$R_1R_2NH$ (II)

wobei $R_1$ und $R_2$ jeweils H oder $C_{1-6}$-Alkyl sind, mit der Maßgabe, daß nur einer von $R_1$ oder $R_2$ H ist; oder $R_1$ und $R_2$, wenn sie zusammengefaßt sind mit dem Stickstoffatom, an das sie geknüpft sind, einen 5- oder 6-gliedrigen Ring bilden, der höchstens zwei Heteroatome enthält; in einem inerten Lösungsmittel, bei einer Temperatur von etwa 25°C bis zum Rückfluß, während 1 bis 24 Stunden und weitere Umsetzung der resultierenden Mischung von Alkylaminomaleat oder Alkylaminofumarat der Formel IIIa und Chloraminosuccinat der Formel IIIb

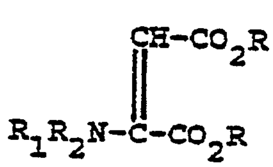

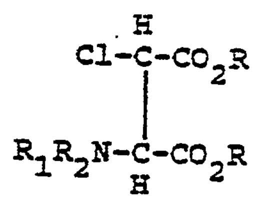

IIIa IIIb

wobei R, $R_1$ und $R_2$, wie oben bei Formel I und Formel II beschrieben sind, mit einem molaren Äquivalent Anilin in einem inerten Lösungsmittel, das eine organische Säure enthält; bei einer Temperatur von 25°C bis 90°C, während 1 bis 24 Stunden; Umsetzung des so gebildeten Anilinofumarats mit einer äquivolaren Menge eines Vilsmeier-Reagenz in Anwesenheit eines Kohlenwasserstofflösungsmittels oder eines chlorierten Kohlenwasserstofflösungsmittels bei einer Temperatur von 40°C bis 110°C, während einer Zeitspanne, die ausreicht, um die Reaktion im wesentlichen zu vervollständigen und ein Dialkylchinolin-2,3-dicarboxylat zu erhalten, und die Hydrolyse dieses Dialkylchinolin-2,3-dicarboxylat zu Chinolin-2,3-dicarbonsäure.

6. Verfahren gemäß Anspruch 5, wobei die organische Säure Essigsäure ist, wobei R für $C_2H_5$ steht; wobei das Amin der Formel II Diethylamin oder Morpholin ist; und wobei es sich bei dem inerten, organischen Lösungsmittel um chlorierten Kohlenwasserstoff, chlorierten, aromatischen Kohlenwasserstoff oder Mischungen derselben handelt.

7. Verfahren gemäß Anspruch 6, wobei es sich bei dem inerten, organischen Lösungsmittel um Ethylendichlorid, Monochlorbenzol, Toluol oder Mischungen derselben handelt; wobei es sich bei dem Vilsmeier-Reagenz-Lösungsmittel um einen Kohlenwasserstoff, chlorierten Kohlenwasserstoff oder Mischungen derselben handelt; und wobei es sich bei dem erwähnten Lösungsmittel um Toluol, Methylenchlorid, Dichlorethan, Orthodichlorbenzol, Chlorbenzol oder Mischungen derselben handelt.

8. Verfahren zur Herstellung von Dialkyloxalacetaten mit der Struktur

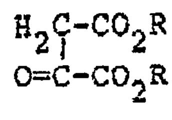

umfassend die Umsetzung eines Dichlorsuccinats der Formel I

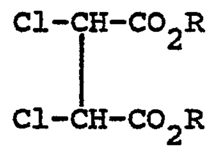

(I)

wobei R für $C_{1-4}$-Alkyl steht mit einem Minimum von 3 molaren Äquivalenten eines Amins der Formel II

$R_1R_2NH$ (II)

wobei $R_1$ und $R_2$ jeweils H oder $C_{1-6}$-Alkyl sind, mit der Maßgabe, daß nur einer von $R_1$ oder $R_2$ H ist; oder $R_1$ und $R_2$, wenn sie zusammengefaßt sind, mit dem Stickstoffatom, an das sie gebunden sind, einen 5- oder 6-gliedrigen Ring bilden, der höchstens 2 Heteroatome enthält; in einem inerten Lösungsmittel bei einer Temperatur von etwa 25°C bis zum Rückfluß, während 1 bis 24 Stunden, und weitere Umsetzung der resultierenden Mischung von Alkylaminomaleat oder Alkylaminofumarat der Formel IIIa und Chloraminosuccinat der Formel IIIb

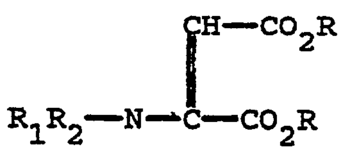

(IIIa)

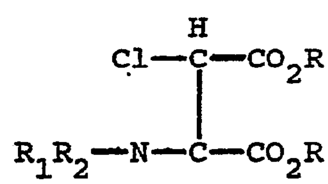

(IIIb)

wobei R, $R_1$ und $R_2$ wie oben bei Formel I und Formel II beschrieben sind, mit wässriger Säure.

9. Verfahren zur Herstellung von Anilinofumarat, umfassend die Umsetzung eines Dichlorsuccinats der Formel I

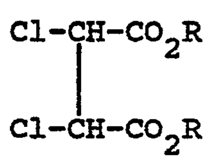

(I)

wobei R für $C_{1-4}$-Alkyl steht, mit einem molaren Äquivalent Anilin in einem inerten, organischen Lösungsmittel und 2 oder mehr molaren Äquivalenten einer wässrigen Base, in Gegenwart eines Phasentransferkatalysators bei einer Temperatur von 20°C bis 90°C, während 1 bis 24 Stunden.

10. Verfahren gemäß Anspruch 9, wobei die wässrige Base 15 bis 50 %-iges, auf Gewichtsbasis, Natriumhydroxid, Kaliumhydroxid oder einer Mischung derselben ist; wobei der Phasentransferkatalysator ein quaternäres Ammoniumsalz ist, bestehend aus solchen Salzen wie n-Butylammoniumchlorid, Benzyltriethylammoniumchlorid oder Mischungen derselben; wobei es sich bei dem organischen Lösungsmittel um einen Kohlenwasserstoff, aromatischen Kohlenwasserstoff, chlorierten, aromatischen Kohlenwasserstoff oder Mischungen derselben handelt; wobei es sich bei dem erwähnten Lösungsmittel um einen chlorierten aromatischen Kohlenwasserstoff, aromatischen Kohlenwasserstoff oder Mischungen derselben handelt, wobei die erwähnte Base 20 bis 50 %-ig, auf Gewichtsbasis, an Natriumhydroxid, Kaliumhydroxid oder Mischungen derselben ist; wobei die Base 2- bis 3 molare Äquivalente beträgt; und wobei die Temperatur 20°C bis 85°C beträgt.

11. Verfahren gemäß Anspruch 10, wobei die wässrige Base 1,1 bis 3,0 molare Äquivalente von 15 bis 50%-igem, auf Gewichtsbasis, Natriumcarbonat, Kaliumcarbonat, oder einer Mischung derselben ist und wobei das quaternäre Ammoniumsalz Tricaprylylmethylammoniumchlorid oder Tributylethylammoniumchlorid ist.